(19) Europäisches Patentamt
European Patent Office
Office européen des brevets

(11) **EP 3 524 285 A1**

(12) **EUROPEAN PATENT APPLICATION**

(43) Date of publication:
**14.08.2019 Bulletin 2019/33**

(51) Int Cl.:
**A61M 1/10** (2006.01)  **A61M 1/12** (2006.01)
**A61F 2/24** (2006.01)  **A61F 2/06** (2013.01)

(21) Application number: **18156158.0**

(22) Date of filing: **09.02.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **Koninklijke Philips N.V.**
**5656 AE Eindhoven (NL)**

(72) Inventors:
• **PELSSERS, Eduard Gerard Marie**
**5656 AE Eindhoven (NL)**

• **HENDRIKS, Cornelis Petrus**
**5656 AE Eindhoven (NL)**
• **BOUMA, Peter Hermanus**
**5656 AE Eindhoven (NL)**
• **JOHNSON, Mark Thomas**
**5656 AE Eindhoven (NL)**
• **VAN DEN ENDE, Daan Anton**
**5656 AE Eindhoven (NL)**
• **VAN DER HORST, Arjen**
**5656 AE Eindhoven (NL)**

(74) Representative: **de Haan, Poul Erik**
**Philips International B.V.**
**Philips Intellectual Property & Standards**
**High Tech Campus 5**
**5656 AE Eindhoven (NL)**

(54) **IMPLANT DEVICE FOR IN-BODY BLOOD FLOW CONTROL**

(57) An implantable blood flow control system has an open passageway defined inside a radially inner wall, behind which is a closed passageway. The radially inner wall comprises electroactive polymer actuator members for providing a variable flow restriction. The flow restriction has an undulating shape so that the volume of the closed passageway may remain constant during actuation of the electroactive polymer actuator members. In this way, the chamber behind the actuator members does not impede free movement of the actuator members. The undulating pattern presents no sharp edges, where blood coagulation could otherwise occur.

FIG. 1

**EP 3 524 285 A1**

## Description

FIELD OF THE INVENTION

This invention relates to implant devices for in-body blood flow control.

BACKGROUND OF THE INVENTION

There is increasing demand for unobtrusive in-body health sensing and treatment systems. In particular, there is a shift from conventional hospital treatment towards unobtrusive vital signs sensor and actuator technologies, centered around the individual, to provide better information about the subject's general health and to provide personalized treatment functions.

Vital signs monitor systems for example help to reduce treatment costs by disease prevention and enhance the quality of life. They may provide improved physiological data for physicians to analyze when attempting to diagnose a subject's general health condition. Vital signs monitoring typically includes monitoring one or more of the following physical parameters: heart rate, blood pressure, respiratory rate and core body temperature.

It is known to provide implantable sensor devices for monitoring physiological parameters, such as blood pressure. While the initial insertion is an invasive procedure, once this is completed, the sensor remains in place for unobtrusive sensing for a prolonged period of time. Thus, implanted sensor technologies are also seen as minimally invasive (in the long term). It is also known to provide implantable actuator devices, the most obvious example being pacemakers.

Implantable devices enable unobtrusive and long term monitoring and treatment of patients with chronic diseases such as heart failure, peripheral artery disease or hypertension.

This invention relates more particularly to the treatment of diseases that originate from, or are the cause of, an imbalance of in-body blood flows. These can in theory be mitigated to a certain extent by correction of in-body flows by increasing or decreasing the hydrodynamic resistance of particular flow channels in a timely manner.

Vascular steal is a decreasing or ceasing blood supply in a part of a vasculature due to the increase of blood flow into another part of the vasculature. For instance, after deployment of a stent in a blood vessel, a sudden lowering of blood flow hydrodynamic resistance occurs, which may induce a sudden lowering of blood flow in another blood vessel. This deprives the affiliated tissue microcirculation of blood. Such an effect can occur for instance in the lower limbs when placing a peripheral artery stent or creating a bypass on an occluded coronary artery or placing a renal stent.

However a sudden increase of in-body flow in itself can cause damage to the downstream tissue as well. For instance, when a stent is placed, a sudden increase of oxygen downstream can damage the tissue that cannot adapt sufficiently fast to the increased oxygen concentration. For instance, it is well known that when too much blood is supplied to a kidney, kidney damage can occur. Myocardial reperfusion injury, after opening a coronary vessel, can be a major contributor to the final infarct size. Additionally, reperfusion injury also occurs after stroke treatment.

There are other examples where blood flow conditions may have an adverse effect. An imbalance in the pumping of the right ventricle (RV) and the left ventricle (LV) of the heart can cause fluid buildup into the lungs which seriously hampers the oxygen uptake by the lungs. A blood pressure in the lower legs and feet which is too high can cause swelling which can create i.e. a thrombosis, whereas a blood supply which is too low can cause diabetic foot and chronic limb ischemia (CLI).

Depending on the disease to be treated, an implanted device may have to operate at least for tens of minutes to days and even months and should preferably be delivered by minimally invasive techniques. Also retrieval, if required, is preferred by minimally invasive techniques. Alternatively the device may be left in the body after the functional time window has passed.

There are particular problems associated with introducing blood flow control within a vessel. In particular, sharp edges or localized regions of low flow may result in blood coagulation. In addition, the power consumption of any implanted actuator should be as low as possible to enable long term operation.

There is therefore a need for an implant which is able to implement a change of in-body blood flow in a timely controlled fashion. There is a need to change flow resistance while minimally disturbing required flows in other parts of the flow system, and which can be implemented continuously or at least in a regular fashion. Clearly, a small form factor is also required.

SUMMARY OF THE INVENTION

The invention is defined by the claims.

According to examples in accordance with an aspect of the invention, there is provided an implantable blood flow control system, comprising:

a radially outer wall;
a radially inner wall;
a first, open, passageway defined inside the radially inner wall,
a second, closed, passageway defined between the radially inner and outer walls, wherein the second, closed, passageway is closed at its ends,
wherein the radially inner wall comprises electroactive polymer actuator members for providing a variable flow restriction, and
wherein the flow restriction provided by the electroactive polymer actuator members has first regions of increased radius of the first, open, passageway and second regions of decreased radius of the first, open, passageway, for reducing variations in the volume of the second, closed, passageway during actuation of the electroactive polymer actuator members.

This implantable system provides an inner, open, passageway which has a shape which is varied by making use of electroactive polymer actuators. On one side of the actuators is the blood flow passageway, and on the other side is a closed chamber. The actuator members form an undulating pattern with the aim of maintaining the volume of closed chamber substantially constant (for example with a volume variation of less than 10% of a non-actuated baseline volume). In this way, the chamber behind the actuator members does not impede free movement of the actuator members; in particular there is no partial vacuum created to resist movement of the actuator members. The undulating pattern preferably presents no sharp edges, where blood coagulation could otherwise occur.

The second passageway is closed so that blood flow does not enter the second passageway, and hence no coagulation of blood may take place in the second passageway.

The system is used for controlling blood flow by providing a controllable flow restriction. There are various diseases related to an imbalanced flow of blood. There is a need to correct this imbalanced flow continuously or at least in regular fashion, ruling out invasive intervention every time when needed. The invention enables flow control in the body at local positions, in autonomous manner and with a device having a small form factor.

The system has a tubular design which may be delivered using catheter based approaches, such as already used for stents and other implants.

The electroactive polymer actuator members for example comprise bending actuators, mounted at their ends to radial supporting walls which connect to the radially outer wall. Some bend inwardly to cause a flow restriction in the open passageway and others bend outwardly, to maintain a near constant volume of the closed chamber.

The radial supporting walls preferably comprise vents so that the chamber areas behind adjacent actuator members are fluidly connected to each other to create a shared volume.

In a first set of examples, the first and second regions are positioned alternately around the circumference of the radially inner wall. In this way, the shape of the open passageway, in cross section perpendicular to a length of the system, has an undulating form, with regions of lower radius and regions of higher radius. There may for example be 8 actuator members so that the open passageway has a generally octagonal shape when not actuated, and a "+" shape when actuated. Generally, there is preferably an even number of actuator members, and there may typically be 6, 8, 10 or 12.

In this set of examples, the system has a length and has a constant cross sectional shape along its length. The flow restriction function is based on control of the surface area of the outer wall of the open passageway.

In a second set of examples, the system has a length and the first and second regions are positioned alternately along the length. In this way, the shape of the open passageway has an undulating form, with regions of lower radius and regions of higher radius at different positions along its length. The regions of lower radius and the regions of higher radius are circles so that there is a more gradual curvature in the undulations (depending on the length of the implantable system). Thus, the first, open, passageway has a generally circular cross sectional shape, with different diameters at different positions along its length when the electroactive polymer actuator members are actuated. The flow restriction function is based on local narrowing of the open passageway.

In all examples, the electroactive polymer actuator members may adopt the least flow restricting configuration when not actuated. Thus, when energy is no longer supplied to the actuator members, the system adopts a configuration of least flow restriction.

The system may further comprise an energy source for providing energy for controlling the electroactive polymer actuator members. Thus, in this case, there is an implanted energy source, with a lifetime sufficient for the intended period of operation.

The system may further comprise a receiver for receiving energy wirelessly for use in controlling the electroactive polymer actuator members. This may be used for directly controlling the actuator members, or for charging a local energy source.

The invention also provides a blood flow control arrangement, comprising:

     an implantable blood flow control system as defined above; and
     a non-implantable energy source for wirelessly powering the implantable blood flow control system.

This arrangement has external powering of the actuator members, and includes the external (i.e. not implanted) power source.

The invention also provides an implantable system, comprising:

     an implantable blood flow control system as defined above; and
     a stent or a flow diverter upstream of the blood flow control system.

A first option provides the combination of a stent and the flow control system. When the stent is introduced, the flow control system enables the flow resistance to be increased more gradually than for a conventional stent procedure. The stent and flow control system may for example be separate implantable devices which are to be placed in series.

However, in another design, the stent is located around the outside of the radially outer wall. This provides an integrated solution which may be implanted as a single component.

A second option provides the combination of a flow diverter and a blood flow control system. The flow diverter may for example be used to steer the flow towards branching vessels.

The invention also provides an implantable system, comprising:

an implantable blood flow control system as defined above;
a blood pressure sensor; and
a controller, wherein the controller is adapted to control the electroactive polymer actuator members in dependence on the sensed blood pressure.

This system enables automated control of the blood flow restriction based on sensing of blood pressure. The sensing may be in the same vessel as the flow control system or it may be in a different vessel, but which is nevertheless affected by the flow restriction.

The blood pressure sensor may for example comprise an electroactive polymer sensor, and hence of the same type as the flow control actuator members. When sensing and actuation may be at the same location, a single electroactive polymer device may be used for both sensing and actuation.

BRIEF DESCRIPTION OF THE DRAWINGS

Examples of the invention will now be described in detail with reference to the accompanying drawings, in which:

Figure 1 shows a system for controlling blood flow;
Figure 2 shows a first example of the implantable actuator;
Figure 3 shows an example of the die shape used for compressing the actuator of Figure 2;
Figure 4 shows a second example of the implantable sensor;
Figure 5 shows an implant which makes use of actuator members in the form of flaps;
Figure 6 shows an implant which makes use of actuator members placed around the outer circumference of a blood vessel to restrict blood flow;
Figure 7 shows an integrated stent and flow control device, with the flow control device based on the design of Figure 4;
Figure 8 shows an example of a separate implantable sensor device;
Figure 9 shows a flow control device and a sensor forming an overall implanted system as well as an external electrical power device; and

Figure 10 shows a device based on the device of Figure 5 which has asymmetry and is combined with a flow diverter element for positioning before the flow control device in the blood vessel.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an implantable blood flow control system in which an open passageway is defined inside a radially inner wall, behind which is a closed passageway. The radially inner wall comprises electroactive polymer actuator members for providing a variable flow restriction. The flow restriction has an undulating shape so that the volume of the closed passageway may remain constant during actuation of the electroactive polymer actuator members. In this way, the chamber behind the actuator members does not impede free movement of the actuator members. The undulating pattern presents no sharp edges, where blood coagulation could otherwise occur.

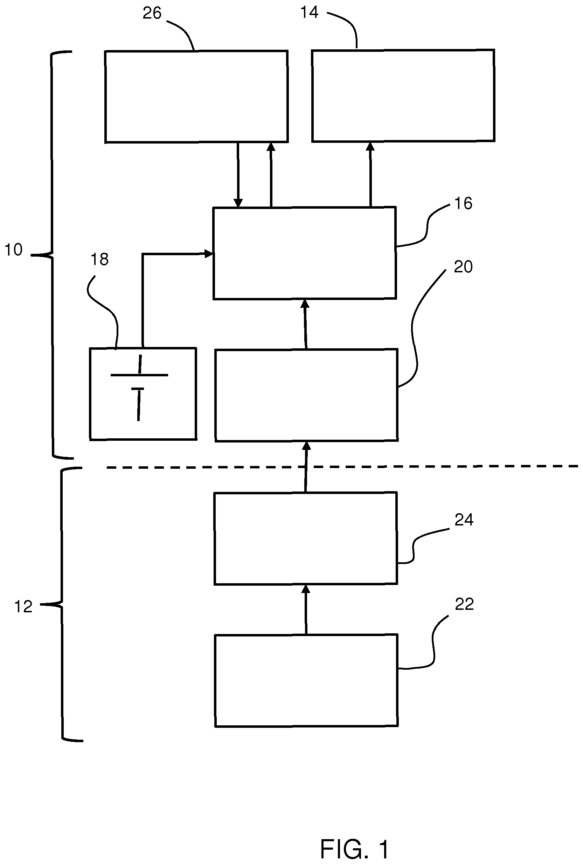

Figure 1 shows a system for controlling blood flow. Different implementations may use different ones of the components shown, as will be clear from the description below. The system has implantable part 10 and an external, non-implantable part 12.

The implantable part comprises an actuator 14 for providing a controllable flow restriction and a controller 16. The controller 16 is powered by an internal power source 18 and/or by receiving power wirelessly using a receiver 20.

The external part has an external power source 22 and a wireless power transmitter 24.

The actuator may be controlled in open loop without any feedback mechanism. However, Figure 1 also shows a sensor 26, such as a blood pressure sensor, which is used as an input for feedback control. The sensor and actuator together form an electroactive polymer sensor-actuator combination.

The system also includes a fixing arrangement (not shown) for fixing the implanted parts of the system in the body.

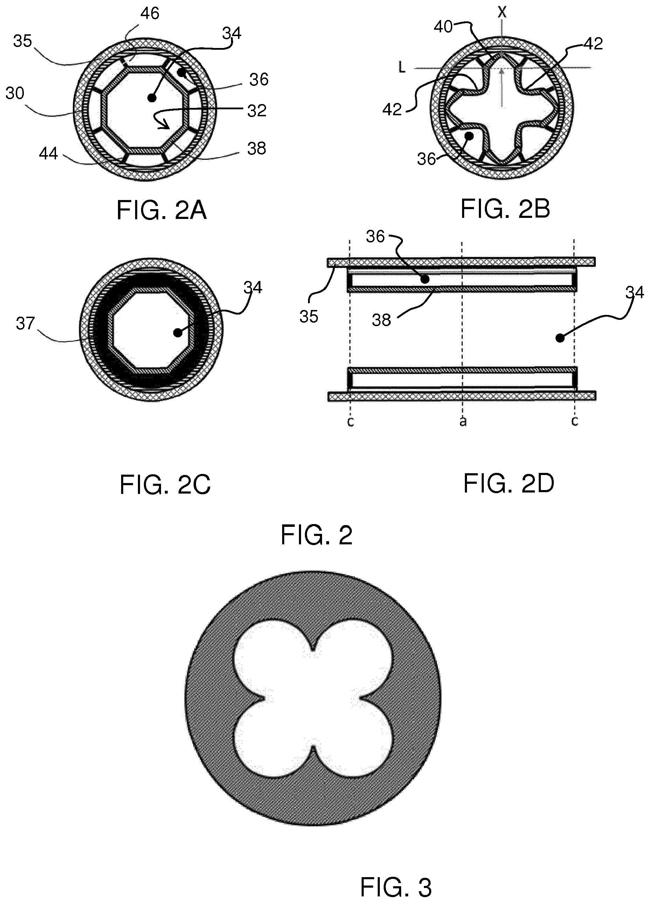

Figure 2 shows a first example of the implantable actuator. The actuator is generally cylindrical, and is for mounting within a blood vessel or artery.

Figure 2A shows a cross section (perpendicular to the length direction) in a non-actuated state at an intermediate position along the length, and Figure 2B shows a cross section (perpendicular to the length direction) in an actuated state for providing a blood flow restriction. Figure 2C shows a cross section at one end. Figure 2D shows a cross section in a plane parallel to the length.

The actuator comprises a radially outer wall 30 and a radially inner wall 32. The actuator is mounted inside an artery or other blood vessel 35. A first, open, passageway 34 is defined inside the radially inner wall 32 and a second, closed, passageway 36 is defined between the radially inner and outer walls 30,32. The second, closed, passageway 36 is closed at its ends by a supporting wall 37 as shown in Figure 2C.

The radially inner wall 32 comprises electroactive polymer actuator members 38 for providing a variable flow restriction.

The flow restriction provided by the electroactive polymer actuator members is seen in Figure 2B. It has first regions 40 of increased radius of the first, open, passageway and second regions 42 of decreased radius of the first, open, passageway 34. This reduces or eliminates variations in the volume of the second, closed, passageway 36 during actuation of the electroactive polymer actuator members.

The first and second regions are positioned alternately around the radially inner wall 32 so that the open passageway has an undulating form in the cross section shown in Figure 2B. The example shows 8 actuator members so that the open passageway 34 has a generally octagonal shape when not actuated (Figure 2A), and a cross ("+") shape when actuated (Figure 2B). Generally, there is preferably an even number of actuator members, and there may typically be 6, 8, 10 or 12. The implanted actuator has a constant cross sectional shape along its length for all actuation (or non-actuation) levels. The individual actuator members thus adopt a portion of a cylindrical shape.

The undulating pattern means that the volume of the closed chamber 36 is substantially constant. For example, for all actuation levels, there is a volume variation of less than 10% from a non-actuated baseline volume.

In this way, the chamber formed by the closed passageway 36 behind the actuator members does not impede free movement of the actuator members, because there is no partial vacuum created to resist movement of the actuator members. The closed chamber 36 for example is filled with a fluid or compressible porous material filled with a fluid.

The electroactive polymer actuator members 38 comprise bending actuators, mounted at their ends to radial supporting walls 44. These connect to the radially outer wall 30 and they have vents 46 so that the chamber areas behind adjacent actuator members 38 are fluidly connected to each other to create a shared volume.

Because in this design the volume (i.e. cross sectional area in the cross section shown) of the closed passageway 36 remains constant, it follows that the volume (and cross sectional area) of the open passageway 34 is also constant. However, actuation results in an increase in circumference, hence the surface area of the device in contact with the blood is increased and hence the hydrodynamic resistance is increased. The surface of the device in contact with the blood remains a smooth surface and hence there are no pockets where blood is not flowing. This is beneficial for preventing an initiation of blood coagulation.

The actuator members are arranged in an asymmetrical fashion so that when actuated they move in a predefined direction (radially inwardly or radially outwardly). Half of the actuator members are oriented in such a manner that they move radially outwardly and half of the actuator members are oriented to move radially inwardly. If all actuator members were arranged to move radially inwardly then the volume of the second passageway 36 would increase, and this would create a vacuum which would counteract the actuator members moving inwardly.

To verify and calculate the increase of the hydrodynamic resistance, a computational flow dynamics software package has been used. The example of Figure 2 was used whereby the displacement was chosen to be 0.24, which is the value of X (change in radial position) /L (effective width of actuator member).

X and L are indicated in Figure 2. The increase in hydrodynamic resistance between a non-actuated and actuated state was calculated to be 19% which is a relevant value for a clinical application.

Since half of the actuator members displace inwardly and the other half displace outwardly, there is almost no force counteracting the displacement. So even weak actuators can be used, giving more freedom in the design.

To provide the bending in the desired direction, the electroactive polymer actuator members may be prestretched. The example of Figure 2 makes use of actuator members with actuation aligned with the plane of the cross section of the device.

As mentioned above, feedback control may make use of a blood pressure sensor. A sensor that can monitor the blood pressure may be based on one or more of the electroactive polymer (EAP) actuator members themselves. By adding two pressure sensors, one proximal to the device and one more distal, the effect of the actuation on the blood flow can be determined directly and used in a feedback loop. Similarly a flow sensor could perform the same function.

To enable delivery of the device into the blood vessel using a catheter, the outer circumference (of the radially outer wall 30) may be folded by pushing the device through a hollow die folding the device such that the external diameter decreases and can fit the inner lumen of the catheter. Figure 3 shows an example of the die shape.

When ejected out of the catheter, the flexible walls of the device may then unfold and since the outer diameter of the device is slightly larger than the blood vessel inner diameter, the device is fixed by pressure onto the blood vessel wall. Typically, the outer radial wall 30 is made of a memory material or a material with spring like properties and thereby when ejected out of the catheter, it will adapt to its original cylindrical form. By increasing the intrusions of the die, a higher folding level can be obtained.

The example of Figure 3 corresponds to an original device diameter which is 23% larger than the inner diameter of the catheter. The die may be tapered in the length direction (not shown) so that a gradual folding occurs.

In the example of Figure 2, the actuation results in a change in the shape of the open passageway 34 in cross section perpendicular to the length direction.

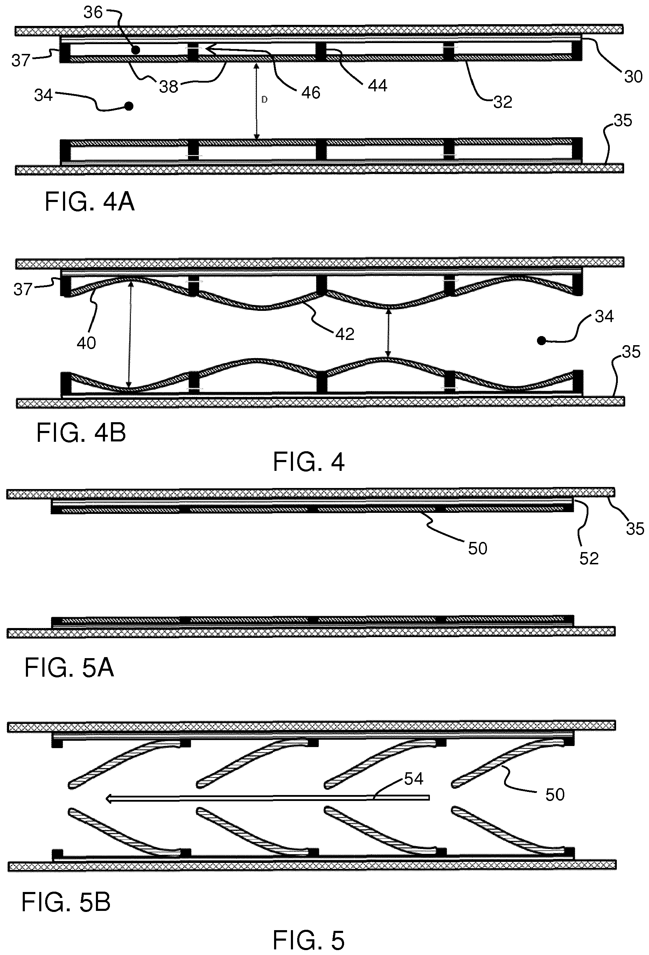

Figure 4 shows a version in which the actuation is aligned with the plane of the length direction of the device. Figure 4A shows a cross section parallel to the length direction for a non-actuated state, and Figure 4B shows the cross section for the actuated state.

The actuator again comprises a radially outer wall 30 and a radially inner wall 32 and the actuator is mounted inside an artery or other blood vessel 35. The first, open, passageway 34 is defined inside the radially inner wall 32 and the second, closed, passageway 36 is defined between the radially inner and outer walls 30,32. The second, closed, passageway 36 is closed at its ends by a supporting wall 37.

The radially inner wall 32 again comprises electroactive polymer actuator members 38 for providing a variable flow restriction. In this example, the actuator members are generally cylindrical, each extending fully around the open passageway 34. However, each one of these actuator members may in fact be formed of multiple sub-members, where all sub-members are actuated in the same way. Thus, the cross section perpendicular to the length direction may correspond to Figure 2A, for example with 8 actuator members present in the cross section. However, all these actuator sub-members are actuated radially in the same direction. The aim is to create a local narrowing (and hence area reduction) of the open passageway. Because of the use of sub-members, the constriction does not create a circular cross sectional shape, but the shape is more rotationally symmetric. For example, the arrangement of Figure 2B has rotational symmetry of order 4 (half the number of actuator members) whereas the arrangement of Figure 4 will have rotational symmetry of order 8 (the number of actuator members).

The open passageway may be considered to have a generally circular cross sectional shape, with different effective diameters at different locations.

The flow restriction provided by the electroactive polymer actuator members is seen in Figure 4B. It again has first regions 40 of increased radius of the first, open, passageway and second regions 42 of decreased radius of the first, open, passageway 34.

There are again side walls 44 and vents 46 so that the different pockets which define the outer closed passageway are connected.

Alternately along the length of the actuator, half of the actuator members move radially inwardly and half move radially outwardly. In this case, there is again an increase in hydrodynamic resistance caused by increased surface area. However, the dominant mechanism is due to the narrowing of the inner lumen diameter of the device. Although the surface of the device in contact with the blood is deformed the surface again remains a smooth surface and there are no stagnant pockets.

The second, closed, passageway is again filled with a fluid or a porous material filled with a fluid.

By approximation the hydrodynamic resistance of the device is proportional to:

$$Rhy = C * \Sigma \left[ \#members / (D)^4 \right]$$

Where Rhy is the hydrodynamic resistance of the device, C is a proportionality parameter, #members is the number of actuator members along the length of the device in actuated or non-actuated state and D is the inner lumen diameter which varies with the state of the actuator members. For example, referring to Figure 4A, when the non-actuated diameter D is 5 mm and when the diameter at the position of two actuator members actuated radially outwardly is 6 mm and at the position of the other two actuator members actuated radially inwardly is 4 mm then the hydrodynamic resistance is by approximation increased by about 46 % as an approximation.

Note that another option is to actuate diametrically opposite actuator members into different states. As a consequence, a curved blood flow will result. If the direction of curvature of adjacent pairs of actuator members is in the other direction, then a meandering flow will result. Such a flow will also result in a reduced flow rate without a change in the overall volume.

For completeness some other examples of flow control device will now be presented, which do not make use of open and closed passageways as in the examples above.

Figure 5 shows an implant for in-vessel control of blood flow which makes use of actuator members in the form of flaps 50. Figure 5A shows the non-actuated configuration and Figure 5B shows the actuated configuration.

These actuator flaps 50 are provided inside a cylindrical tube 52 which functions as a substrate. They are only fixed to the substrate 52 on one end. When actuated, the actuator members bend inwardly. The blood flow direction is shown by arrow 54. In this case the increase in hydrodynamic resistance is caused by a blocking action of the EAP flaps similar to the action of natural valves in the cardiovascular system.

Behind the flaps 50, a kind of circulating flow can occur and blood flow velocities can be rather low which may induce coagulation. By use of state-of-the-art computational flow dynamics models the 3D geometry and the blood velocities can be simulated. With this tool, optimized shape and dimensions of the actuator members can be realized for the diverse blood vessels. In a Poiseuille flow profile, the velocity on the wall is zero, but by diffusion the net flow of particular molecules and biological cells is not zero. In circulating flow, the average net flow of molecules and cells can be rather low. Unintentional backflow (from left to right in Figure 5B) is prevented by passive valve closing due to the backflow.

The blood flow restricting device can operate in two manners:

(i) If the blood flow is from left to right, the blood flow will support the bending of the actuator members. When the blood flow restriction is not needed any more, the force that relaxes the actuator members back to their original non-actuated state should be large enough to withstand the force executed on the actuator member by the blood flow. Due to the contraction of the heart, there is pulsed pressure and the actuator members could be relaxed when the pressure is the lowest.

(ii) If the blood flow is from right to left, the force bending the actuator members should be larger than the force exerted on the actuator members by the blood flow. Here bi-stable actuator designs may be beneficial since energy is only required when the EAP state is switched. Bi-stable electroactive polymer actuators are for example discussed in WO 2016/193412.

When the blood flow restriction is not needed any more, the force that relaxes the actuator members back to their original none-actuated state is supported by the force exerted by the blood flow. Due to the contraction of the heart there is pulsed pressure and the actuator members could be relaxed when the pressure is the highest.

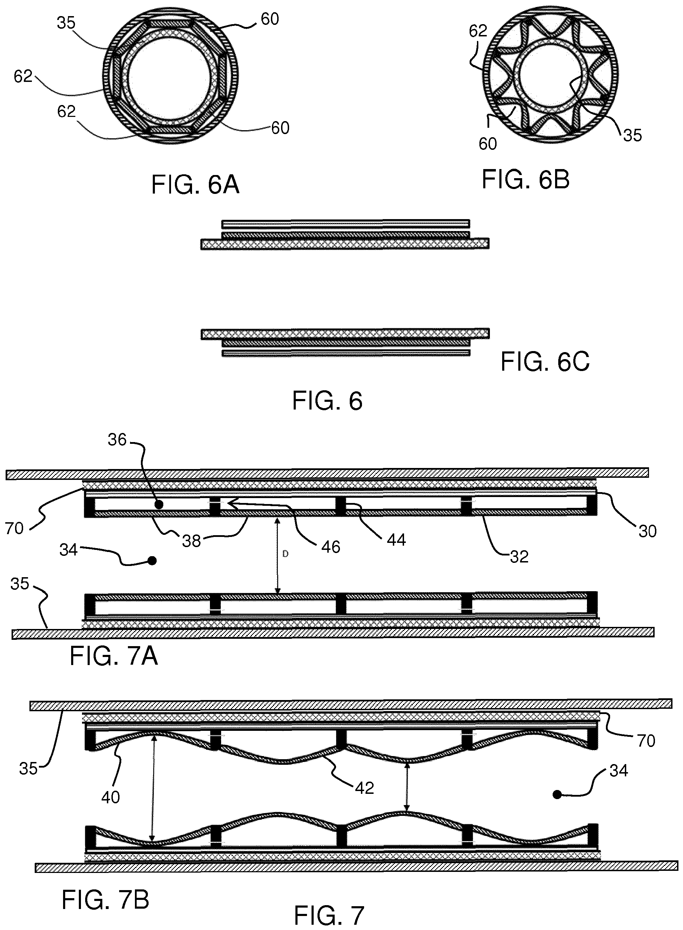

Figure 6 shows an implant for in-vessel control of blood flow which makes use of actuator members placed around the outer circumference of a blood vessel 35 to restrict blood flow.

Figure 6A shows a cross section (perpendicular to the length direction) in a non-actuated state and Figure 6B shows a cross section (perpendicular to the length direction) in an actuated state for providing a blood flow restriction. Figure 6C shows a cross section in a plane parallel to the length.

Actuator members 60 are located on a substrate 62 in the form of a cylindrical cuff. When actuated, the flexible blood vessel wall is deformed and, at the position of the cuff, the inner diameter of the blood vessel is decreased and hence the hydrodynamic resistance is increased. As such, the blood vessel is contracted at the position of the cuff but the blood vessel wall remains smooth. This is again beneficial for avoiding the initiation of blood coagulation. An application for such a cuff device is during coronary bypass surgery, where the sudden opening of the bypass graft may also lead to reperfusion injury. A cuff around the bypass vessel is able to control this.

Note that the contraction of the blood vessel is depicted schematically in Figure 6. Depending on the level of actuation and the diameter and elasticity of the blood vessel, the circumference may be deformed somewhat due to the fact that the actuator members apply a force at certain discrete positions to the blood vessel.

If there are nerves located on the surface of the blood vessel, actuating the cuff can apply a pressure on these nerves and thereby control nerve activity. For example, renal nerves are located on the outer surface of the renal arteries, and they are instrumental in transferring nerve signals to the kidneys, which in turn influence blood pressure. The compression of nerves should be limited to avoid a pain sensation. Another possible application is the reversible control of the baroreceptors (e.g. Nervus Vagus) which are important in autoregulation of the blood pressure.

One use of the flow control device is to provide control following implantation of a stent. Thus, one aspect is to provide an electroactive polymer actuator device for providing a controllable flow restriction in combination with a stent. State-of-the-art stents unfold in a relatively fast manner thereby opening up a stenosis or chronic total occlusion (CTO) so fast that two detrimental effects can occur.

First, a sudden increase of blood flow will increase the oxygen concentration in the tissue supplied by the blood vessel. This sudden increase of oxygen can damage the tissue; the tissue requires time to adapt to the new conditions it is exposed to. For instance, patients have been cooled to minimize this adverse effect. However, this is a cumbersome procedure.

Second, another effect of a sudden opening of a stenosis or CTO it that suddenly the available blood is distributed in a new manner. Other blood vessels, nearby the blood vessel being stented, are deprived from blood (vascular steal). Consequently the tissue that is supplied with blood by these other blood vessels can be damaged as well. For instance, such an effect can occur in the lower limbs when placing a peripheral artery stent or creating a bypass on an occluded coronary artery or placing a renal stent.

The controllable flow control device may be used to gradually and slowly open up the stenosis or CTO or even reverse the opening up to regulate the blood in a balanced fashion.

A combination of a state-of-the-art stent and a flow restrictor in series could be used. Any of the flow control devices above may be used as flow restrictor in such an arrangement. When deploying and unfolding the state-of-the-art stent the flow restrictor is in a closed configuration and then slowly starts to open. Thus, one aspect provides an implantable blood flow control system, comprising a stent; and a flow control device which comprises an electroactive polymer actuator arrangement for providing a variable flow restriction.

The stent and flow device may instead be formed as an integrated device.

Figure 7 shows an integrated stent and flow control device, with the flow control device based on the design of Figure 4.

Figure 7 shows a stent 70 around the outside of the flow control device of Figure 4. Figure 7A shows the non-actuated state and Figure 7B shows the actuated state, which is used immediately after device implantation. The flow is increased in a timely and reversible manner for widening a narrowed blood vessel. The stent 70 is a grid like structure which when deployed will automatically open up. When the stent is deployed the actuator members are controlled to close the blood vessel. Subsequently, the actuator members are controlled to decrease the hydrodynamic resistance of the device gradually over time.

In all examples above, for safety reasons for instance when the battery is empty and thereby the actuation stops, the non-actuated stated should be such that the device does not block blood flow.

The examples above describe only the actuator design. As mentioned with reference to Figure 1, the overall system may include a sensor.

In certain cases, the blood flow control needs to be carried out on the basis of blood pressure. For instance, as mentioned above, an imbalance in the pumping of the right ventricle (RV) and the left ventricle (LV) of the heart can cause fluid buildup into the lungs which seriously hampers the oxygen uptake by the lungs. This can be monitored by sensing the pressure in the vena cav. By regulating the flow of the vena cava into the RV, the imbalance can be corrected. Since such an imbalance is determined by the pumping performance of RV and LV that can change over time, this regulation has to be done on the basis of a feedback control system, hence by measuring the pressure in the vena cava. Since flow the control device and sensor can be positioned in the same location, one or more of the electroactive polymer actuator members may also be used for sensing the blood pressure.

In another example, as also mentioned above, there is a need to prevent vascular steal by distributing blood flow in a controlled manner over a blood vessel which was previously (partially) blocked and another blood vessel which is not blocked. When the blocked vessel is stented, the blood flow distribution changes abruptly which is detrimental for the tissue which is (also) served with blood by the previously blocked vessel (a too sudden increase in oxygen) and the tissue that is supplied by the non-blocked vessel abruptly receives less blood and thereby experiences a sudden oxygen decrease. Hence the need for a slowly changing blood distribution so that tissue can adapt to the new situation. Moreover since over time hydrodynamic resistance of blood vessel can change, there is a need to adapt the blood flow distribution over time. In this example the sensor is not integrated with the blood flow control device but positioned in the other vessel. Consequently a separate device is required for sensing.

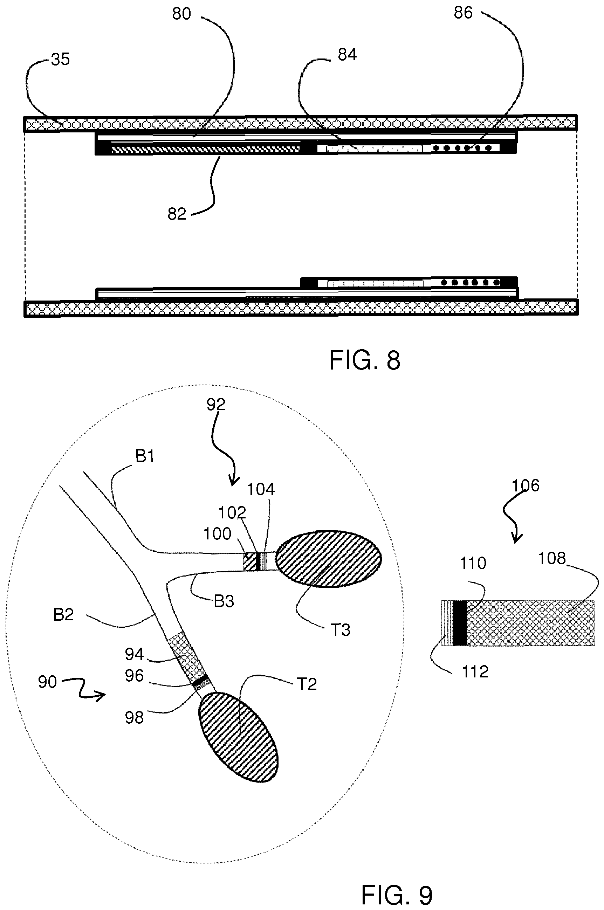

Figure 8 shows an example of a separate implantable sensor device with an electronic circuit and receiver/transmitter coil. The sensor comprises a cylindrical tube 80 within the blood vessel wall 35, having an EAP sensor 82, an electronic circuit 84 and a coil 86 which functions as a power receiving device and a sensor signal transmission device.

To enable blood flow pressure sensing with an electroactive polymer device, use may be made of a sensor-actuator which allows simultaneous sensing and actuation. For an IMPC sensor-actuator, this can be achieved by measuring the impedance of the outer electrodes separately to the actuation voltage or adding a high frequency signal to the quasi-dc actuation signal. The approach of combining a DC actuation signal with a high frequency superposed AC signal for sensing is described in detail in WO 2017/036695.

This AC ripple is provided by the electronic circuit 84 and the same circuit monitors the electrical properties of the EAP element which are a measure for the blood flow pressure. This circuit 84 receives electrical energy via the electrical coil 86 from a remote power source and via this same coil sends the measured values to an actuator device, in this case physically separated from the sensor device. Such an electronic circuit 84 may be provided on a small printed circuit board with appropriate electronic components or may be integrated in one ASIC (application specific integrated circuit). Obviously a separate sensor based on another technology may also be employed to measure pressure or flow.

Figure 9 shows a flow control device 90 and a separate sensor 92. The flow control device has an actuator 94 as described above combined with an electronic control circuit 96 and a receiver coil 98.

A blood vessel B1 bifurcates into two bifurcated vessels B2 and B3. Blood vessel B2 supplies blood to the distal tissue T2 and blood vessel B3 supplies blood to the distal tissue T3.

The flow control device 90 is within vessel B2. The sensor is within vessel B3 and comprises a sensor 100, electronic circuit 102 and a coil 104 for receiving power and also for transmitting the sensor measurements. The sensor may be an electroactive polymer sensor, but another sensor can be used based on another sensing principle. However if the sensor is at the same location as the actuators, one EAP element can be used as a sensor as well as an actuator.

Figure 9 also shows an external electrical power device 106 consisting of a battery 108, an electronic circuit 110 and an electrical transmitter coil 112.

In one example of use of this system, at first the blood vessel B2 has a severe stenosis or even a chronic total occlusion. To remove this blockage in a controllable manner first the sensor device 92 is placed in blood vessel B3.

Secondly an adaptive stent 90 (including an electronic circuit and a receiver coil) is placed at the blockage position. The adaptive stent is in the closed state or at least in such a state that the blood flowing through blood vessel is similar to before the stent was placed.

Subsequently, the electronic circuit 110 of the external power source 106 is switched on and provides via its transmitter coil electrical energy to the electronic circuits in the body. The electronic circuit of the sensor device provides an AC ripple to the EAP element of the sensor and subsequently the same electronic circuit measures the electrical property of the EAP element which is a measure for the blood pressure.

Immediately, the values of these measurements are transmitted via the electrical coil 104 to the receiver coil 98 of the adaptive stent 90 and provided to the electronic circuit 96. The circuit starts to slowly open up the adaptive stent in blood vessel B2, by applying a DC voltage to the EAP actuator members. In this manner, the distal tissue T2 can adapt to the increased blood flow without being damaged by a sudden increase in oxygen concentration. However when the received blood pressure value of blood vessel B3 drops below a preset threshold, the rate of opening up the adaptive stent may be further slowed down to prevent too fast decrease of blood flow in blood vessel B3 so that the distal tissue T3 is not too suddenly deprived from too much blood flow/oxygen.

Finally the opening up of the adaptive stent is stopped when the blood pressure in blood vessel B3 reaches a preset minimum (a second threshold value) or when the stent is fully open.

Over longer times the blood vessels may change, for instance arteriosclerosis changes the hydrodynamic resistance of a blood vessel and/or collateral may redistribute the blood. This may invoke the need for a new balance. Via the external power source, a new set of thresholds can be sent to the electronic circuit of the adaptable stent 90.

In this example, there is an external power source, however it may be chosen to integrate a battery as well into the implantable devices; this will depend on the capacity of a miniature battery and the required functional time of the devices.

In the example, the electrical coils are positioned in an additional cylindrical shaped device connected to the actuator or sensor elements. Alternatively the electrical coils are integrated into the outer cylindrical tube of the sensor or actuator design.

The examples above enable analog control of a level of flow restriction. Alternatively if two states of hydrodynamic resistance are sufficient, the use of bi-stable EAP actuators can be considered, as described in WO 2016/193412. The advantage is that that only energy is required to switch from one to the other state.

Instead of using a battery as an implanted power source, an energy harvesting device can be used which extracts energy from the human body and transforms this energy into electricity. As such, the devices can be operated for prolonged time.

The flow control device may have an external diameter in its unfolded state that is substantially larger than the internal diameter of a diseased blood vessel. In this way, the blood vessel may be widened locally to increase blood flow and when less blood flow is required the flow control device as described above can be actuated to increase the hydrodynamic resistance.

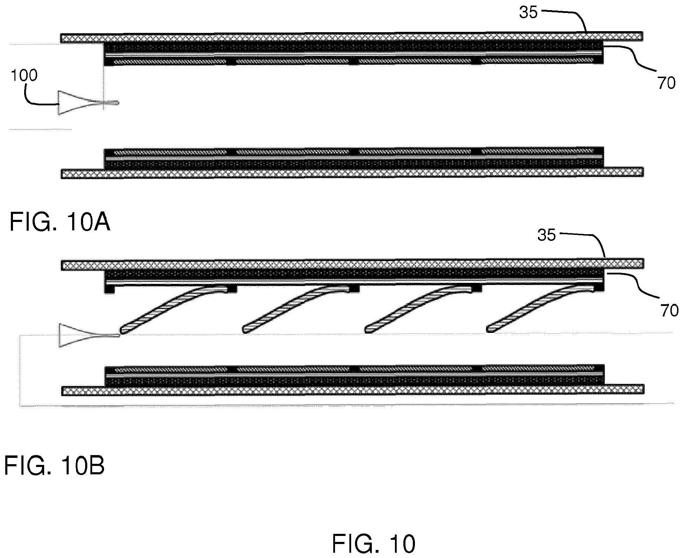

The devices described above all have some rotational symmetry. Figure 10 shows a device based on the device of Figure 5 which has asymmetry and is combined with a flow diverter element 100 for positioning before the flow control device in the blood vessel. This provides a single device which actively regulates and controls blood flow in two vessels separately (for example in both directions after a bifurcation branch), as well as the total supply to both branches.

The flow diverter is used to steer the flow towards branching vessels. Figure 10A shows the non-actuated state and Figure 10B shows actuator members on one side of the device operated to their full actuated state, blocking the flow to one side and allowing normal flow to the other side of the flow diverter.

The flow diverter may be applied to any other asymmetrically controllable design. For example, the designs of Figures 2 and 4 may be actuated in an asymmetric way to provide different flow restriction functions at different sides of a flow diverter.

Thus, another aspect provides an implantable blood flow control system, comprising a flow diverter; and a flow control device downstream of the flow diverter which comprises an electroactive polymer actuator arrangement for providing a variable flow restriction. The flow restriction is preferably controllable to adopt an asymmetric profile so that flows on opposite sides of the flow diverter are subjected to different flow restrictions.

As mentioned above, there are various options for wireless connection to the implanted sensor, either to provide a remote source of power, or to provide a communication channel or both.

In general an implant, whether passive or active, may be powered in many ways. Depending on the functionality and operation mode of the implant, different requirements for the energy source are present.

For a continuous active function, such as a requirement for active mechanical actuation in order to generate an output signal, there is a higher energy requirement than for a passive temporally limited (e.g. on-demand) function, such as the occasional read out of an active sensor or occasional change to an stable actuator. However in both cases, there is a need for either a wired connection to a local power source, or a wireless coupling to a power transmitter.

Delivering electrical power to medical implants for powering or communication is a topic which is well-described in literature.

Comprehensive reviews of power aspects for implantable medical devices are given in B. A. Achraf, A. B. Kouki and C. Hung, "Power Approaches for Implantable Medical Devices," sensors, no. 28889-28914; doi:10.3390/s151128889, 2015, J. Lee, J. Jang and Y.-K. Song, "A review on wireless powering schemes for implantable microsystems in neural engineering applications," Biomed Eng Letters, no. DOI 10.1007/s13534-016-0242-2, pp. 6:205-215, 2016, A. Kim, M. Ochoa, R. Rahim and B. Ziaie, "New and Emerging Energy Sources for Implantable Wireless Microdevices," IEEE: SPECIAL SECTION ON NANOBIOSENSORS, no. 10.1109/ACCESS.2015.2406292, 2014, and K. N. Bocan and E. Sejdi'c, "Adaptive Transcutaneous Power Transfer to Implantable Devices: A State of the Art Review," sensors, vol. 16, no. doi:10.3390/s16030393, p. 393, 2016.

Any of these solutions may be used to power the implant, and some approaches will be discussed below.

A first approach is to provide a wired power source as part of the implant. A wired power source may be an ordinary battery (non-rechargeable or rechargeable) such as shown as 18, directly connected to the implant or to its operating electronics. However, since implants usually will be worn over a long period of time, a high capacity and high energy density battery would be of benefit. The power density of (re-chargeable) batteries is expected to grow further making them increasingly suitable for long term monitoring functions.

Instead of conventional batteries, bio-fuel cells or nuclear batteries may be applicable. Another alternative power source, which is very similar to a battery, is a super capacitor, which is a capacitor having an extremely high capacitance and a very low self-discharge characteristic.

Energy harvesters may instead be used to operate any implant. Accordingly a power generator could for example be operated by human body energy such as motion of an extremity but also motion of an inner organ or any dynamics resulting from a fluid flow (blood in an artery) or gas (air in a lung). The power generator may be able to store energy in a super capacitor or re-chargeable battery, and/or be able to directly operate an implant.

An energy harvester does not necessarily need to be in close vicinity to the implant itself but could also be spatially separated. A wired connection may be used between them. Also in the field of energy harvesters, efforts are being made to make them smaller and more efficient in order to make them more attractive as an internal (and everlasting) energy source for medical devices.

Wireless energy transmission systems may be classified according to the physical coupling mechanism, which can be either capacitive, inductive (magnetic) or electromagnetic. All three mechanisms have their own pros and cons and preferred applications. In general, the performance of each approach depends very much on specific boundary conditions such as e.g. the size of the transmitter- and receiver-element (which can be a plate, an inductor or an antenna) and the distance and medium between both elements, as well as their orientation with respect to each other.

An additional smart feature of all wireless power systems is the intrinsic ability of a bidirectional data communication between a transmitter and a receiver.

In applications where low energy levels at short distances need to be transmitted, capacitive coupling may be used. Low to medium power levels at medium to long range may be preferably realized via an electromagnetic coupling. Highest power levels at short distances may be transmitted via an inductive coupling, making use of magnetic fields.

When using sensor feedback, a most basic approach only enables sensor data to be gathered when the external controller is present. However, using such a wireless powering technique would not necessarily imply the need to wear such a transmitter continuously to perform the intended use of the implant. For example, an implant may only need to be operated during certain treatments (in e.g. a hospital) or it may only need to be activated at predefined moments in time (e.g. morning, afternoon, evening).

An alternative use case would be to use such a wireless transmitter overnight, to charge an implanted power source, which would be used to operate an implant during the day. This is a hybrid approach where there is a local energy supply so sensor data can be gathered and stored in memory without an external controller in place, but it has a short duration so needs recharging periodically.

The implanted wireless receiver unit and the implanted sensor and/or actuator may be spatially separated from each other. For example, the receiving element, e.g. a receiver inductance may be located directly underneath the skin, in order to realize a strong coupling between the transmitter and receiver and thus to maximize the energy transmission efficiency and to minimize the charging time of an implanted battery. Of course, this would require a more involved implantation procedure than if the implanted elements are fully integrated into the stent (or other support structure).

There are also options which do not rely on electrical energy to realize a wireless energy transmission system, in particular making use of optical, ultrasonic or mechanical pressure waves.

As discussed above, a controller performs the data processing. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

As mentioned above, the sensor may be implemented using an electroactive polymer (EAP) device. EAPs are an emerging class of materials within the field of electrically responsive materials. EAPs can work as sensors or actuators and can easily be manufactured into various shapes allowing easy integration into a large variety of systems.

Materials have been developed with characteristics such as actuation stress and strain which have improved significantly over the last ten years. Technology risks have been reduced to acceptable levels for product development so that EAPs are commercially and technically becoming of increasing interest. Advantages of EAPs include low power, small form factor, flexibility, noiseless operation, accuracy, the possibility of high resolution, fast response times, and cyclic actuation.

The improved performance and particular advantages of EAP material give rise to applicability to new applications. An EAP device can be used in any application in which a small amount of movement of a component or feature is desired, based on electric actuation or for sensing small movements.

The use of EAPs enables functions which were not possible before, or offers a big advantage over common sensor / actuator solutions, due to the combination of a relatively large deformation and force in a small volume or thin form factor, compared to common actuators. EAPs also give noiseless operation, accurate electronic control, fast response, and a large range of possible actuation frequencies, such as 0 - 1MHz, most typically below 20 kHz.

Devices using electroactive polymers can be subdivided into field-driven and ionic-driven materials.

Examples of field-driven EAPs include Piezoelectric polymers, Electrostrictive polymers (such as PVDF based relaxor polymers) and Dielectric Elastomers. Other examples include Electrostrictive Graft polymers, Electrostrictive paper, Electrets, Electroviscoelastic Elastomers and Liquid Crystal Elastomers.

Examples of ionic-driven EAPs are conjugated/conducting polymers, Ionic Polymer Metal Composites (IPMC) and carbon nanotubes (CNTs). Other examples include ionic polymer gels.

Field-driven EAPs are actuated by an electric field through direct electromechanical coupling. They usually require high fields (tens of megavolts per meter) but low currents. Polymer layers are usually thin to keep the driving voltage as low as possible.

Ionic EAPs are activated by an electrically induced transport of ions and/or solvent. They usually require low voltages but high currents. They require a liquid/gel electrolyte medium (although some material systems can also operate using solid electrolytes).

Both classes of EAP have multiple family members, each having their own advantages and disadvantages.

A first notable subclass of field driven EAPs are Piezoelectric and Electrostrictive polymers. While the electromechanical performance of traditional piezoelectric polymers is limited, a breakthrough in improving this performance has led to PVDF relaxor polymers, which show spontaneous electric polarization (field driven alignment). These materials can be pre-strained for improved performance in the strained direction (pre-strain leads to better molecular alignment). Normally, metal electrodes are used since strains usually are in the moderate regime (1-5%). Other types of electrodes (such as conducting polymers, carbon black based oils, gels or elastomers, etc.) can also be used. The electrodes can be continuous, or segmented.

Another subclass of interest of field driven EAPs is that of Dielectric Elastomers. A thin film of this material may be sandwiched between compliant electrodes, forming a parallel plate capacitor. In the case of dielectric elastomers, the Maxwell stress induced by the applied electric field results in a stress on the film, causing it to contract in thickness and expand in area. Strain performance is typically enlarged by pre-straining the elastomer (requiring a frame to hold the pre-strain). Strains can be considerable (10-300%). This also constrains the type of electrodes that can be used: for low and moderate strains, metal electrodes and conducting polymer electrodes can be considered, for the high-strain regime, carbon black based oils, gels or elastomers are typically used. The electrodes can again be continuous, or segmented.

A first notable subclass of ionic EAPs is Ionic Polymer Metal Composites (IPMCs). IPMCs consist of a solvent swollen ion-exchange polymer membrane laminated between two thin metal or carbon based electrodes and requires the use of an electrolyte. Typical electrode materials are Pt, Gd, CNTs, CPs, Pd. Typical electrolytes are Li+ and Na+ water-based solutions. When a field is applied, cations typically travel to the cathode side together with water. This leads to reorganization of hydrophilic clusters and to polymer expansion. Strain in the cathode area leads to stress in rest of the polymer matrix resulting in bending towards the anode. Reversing the applied voltage inverts bending. Well known polymer membranes are Nation® and Flemion®.

Another notable subclass of Ionic polymers is conjugated/conducting polymers. A conjugated polymer actuator typically consists of an electrolyte sandwiched by two layers of the conjugated polymer. The electrolyte is used to change oxidation state. When a potential is applied to the polymer through the electrolyte, electrons are added to or removed from the polymer, driving oxidation and reduction. Reduction results in contraction, oxidation in expansion.

In some cases, thin film electrodes are added when the polymer itself lacks sufficient conductivity (dimension-wise). The electrolyte can be a liquid, a gel or a solid material (i.e. complex of high molecular weight polymers and metal salts). Most common conjugated polymers are polypyrrole (PPy), Polyaniline (PANi) and polythiophene (PTh).

An actuator may also be formed of carbon nanotubes (CNTs), suspended in an electrolyte. The electrolyte forms a double layer with the nanotubes, allowing injection of charges. This double-layer charge injection is considered as the primary mechanism in CNT actuators. The CNT acts as an electrode capacitor with charge injected into the CNT, which is then balanced by an electrical double-layer formed by movement of electrolytes to the CNT surface. Changing the charge on the carbon atoms results in changes of C-C bond length. As a result, expansion and contraction of single CNT can be observed.

For the sensing functionality, the use of capacitance change is one option, in particular in connection with an ionic polymer device. For field driven systems, a capacitance change can also be measured directly or by measuring changes in electrode resistance as a function of strain.

Piezoelectric and electrostrictive polymer sensors can generate an electric charge in response to applied mechanical stress (given that the amount of crystallinity is high enough to generate a detectable charge). Conjugated polymers can make use of the piezo-ionic effect (mechanical stress leads to exertion of ions). CNTs experience a change of charge on the CNT surface when exposed to stress, which can be measured. It has also been shown that the resistance of CNTs change when in contact with gaseous molecules (e.g. $O_2$, $NO_2$), making CNTs usable as gas detectors.

Sensing may also be based on force measurements and strain detection. Dielectric elastomers, for example, can be easily stretched by an external force. By putting a low voltage on the sensor, the strain can be measured as a function of voltage (the voltage is a function of the area).

The invention may be applied to stents as mentioned above, but also to stent grafts, heart valves, coronary arterial bypass grafts and shunts.

Another application of restricting blood flow is the controlled starvation of a tumor, as long as the blood flow supply is controlled by a rather low number of arteries. If the tumor is like a sponge in healthy tissue, with critical function, blood flow restriction may cause serious adverse effects to the healthy tissue. It is speculated that a slow starvation may prevent a trigger to the tumor to initiate defensive mechanisms such as releasing circulating tumor cells (CTC's) and angiogenesis, while a sudden starvation would lead to defensive actions such as CTC release.

Another application may be the control of nerve activity as mentioned above, such as the reversible control of the baroreceptors (e.g. Nervus Vagus).

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

1. An implantable blood flow control system, comprising:

   a radially outer wall (30);
   a radially inner wall (32);
   a first, open, passageway (34) defined inside the radially inner wall,
   a second, closed, passageway (36) defined between the radially inner and outer walls, wherein the second, closed, passageway is closed at its ends,
   wherein the radially inner wall comprises electroactive polymer actuator members (38) for providing a variable flow restriction, and
   wherein the flow restriction provided by the electroactive polymer actuator members has first regions (40) of increased radius of the first, open, passageway and second regions (42) of decreased radius of the first, open, passageway, for reducing variations in the volume of the second, closed, passageway during actuation of the electroactive polymer actuator members.

2. A system as claimed in claim 1, wherein the electroactive polymer actuator members (38) comprise bending actuators, mounted at their ends to radial supporting walls which connect to the radially outer wall.

3. A system as claimed in claim 2, wherein the radial supporting walls comprise vents (46).

4. A system as claimed in claim 1, 2 or 3, wherein the first and second regions are positioned alternately around the circumference of the radially inner wall (32).

5. A system as claimed in claim 4, wherein the system has a length and has a constant cross sectional shape along its length.

6. A system as claimed in claim 1, 2 or 3, wherein the system has a length and the first and second regions are positioned alternately along the length.

7. A system as claimed in claim 6, wherein the first, open, passageway (34) has a generally circular cross sectional shape, with different diameters at different positions along its length when the electroactive polymer actuator members are actuated.

8. A system as claimed in any preceding claim, wherein the electroactive polymer actuator members adopt the least flow restricting configuration when not actuated.

9. A system as claimed in any preceding claim, further comprising an energy source (18) for providing energy for controlling the electroactive polymer actuator members.

10. A system as claimed in any preceding claim, further comprising a receiver (20) for receiving energy wirelessly for use in controlling the electroactive polymer actuator members.

11. A blood flow control arrangement, comprising:

   an implantable blood flow control system as claimed in claim 10; and
   a non-implantable energy source (220 for wirelessly powering the implantable blood flow control system.

12. An implantable system, comprising:

   an implantable blood flow control system as claimed in any one of claims 1 to 10; and

a stent (70), or a flow diverter (100) upstream of the blood flow control system.

13. A system as claimed in claim 12, comprising a stent (70) which is located around the outside of the radially outer wall.

14. An implantable system, comprising:

   an implantable blood flow control system as claimed in any one of claims 1 to 10;
   a blood pressure sensor; and
   a controller, wherein the controller is adapted to control the electroactive polymer actuator members in dependence on the sensed blood pressure.

15. A system as claimed in claim 14, wherein the blood pressure sensor comprises an electroactive polymer sensor.

FIG. 1

FIG. 2A

FIG. 2B

FIG. 2C

FIG. 2D

FIG. 2

FIG. 3

FIG. 4A

FIG. 4B

FIG. 4

FIG. 5A

FIG. 5B

FIG. 5

FIG. 6A

FIG. 6B

FIG. 6C

FIG. 6

FIG. 7A

FIG. 7B

FIG. 7

FIG. 8

FIG. 9

35

100

70

FIG. 10A

35

70

FIG. 10B

FIG. 10

Europäisches
Patentamt

European
Patent Office

Office européen
des brevets

# EUROPEAN SEARCH REPORT

Application Number

EP 18 15 6158

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2013/178750 A1 (SHEEHAN DAVID M [US] ET AL) 11 July 2013 (2013-07-11) * paragraphs [0178] - [0191]; figures 15A,B * | 1-15 | INV.<br>A61M1/10<br>A61M1/12<br>A61F2/24<br>A61F2/06 |
| A | US 2015/133721 A1 (ROTH MICHAEL D [US]) 14 May 2015 (2015-05-14) * electroactive polymer 210;sleeve 200 about a body part 12; vein 300; paragraphs [0020] - [0024]; figures 2,3 * | 1-15 | |
| A | US 2016/144091 A1 (BREEDON PHILIP [GB] ET AL) 26 May 2016 (2016-05-26) * the whole document * * paragraphs [0077] - [0079]; figures 1d,2a * | 1-15 | |
| A | US 2004/230090 A1 (HEGDE ANANT V [US] ET AL) 18 November 2004 (2004-11-18) * paragraphs [0157] - [0165]; figures 2,3 * | 1-15 | |

|  | TECHNICAL FIELDS SEARCHED (IPC) |
|---|---|
|  | A61M<br>A61F |

The present search report has been drawn up for all claims

| Place of search | Date of completion of the search | Examiner |
|---|---|---|
| The Hague | 12 July 2018 | Van Veen, Jennifer |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EP 3 524 285 A1

**ANNEX TO THE EUROPEAN SEARCH REPORT
ON EUROPEAN PATENT APPLICATION NO.**

EP 18 15 6158

This annex lists the patent family members relating to the patent documents cited in the above-mentioned European search report.
The members are as contained in the European Patent Office EDP file on
The European Patent Office is in no way liable for these particulars which are merely given for the purpose of information.

12-07-2018

| Patent document cited in search report | | Publication date | Patent family member(s) | | Publication date |
|---|---|---|---|---|---|
| US 2013178750 | A1 | 11-07-2013 | US 2013178750 A1 | | 11-07-2013 |
| | | | WO 2013096548 A1 | | 27-06-2013 |
| US 2015133721 | A1 | 14-05-2015 | NONE | | |
| US 2016144091 | A1 | 26-05-2016 | EP 3007742 A1 | | 20-04-2016 |
| | | | ES 2659975 T3 | | 20-03-2018 |
| | | | US 2016144091 A1 | | 26-05-2016 |
| | | | WO 2014199167 A1 | | 18-12-2014 |
| US 2004230090 | A1 | 18-11-2004 | US 2004230090 A1 | | 18-11-2004 |
| | | | US 2007213579 A1 | | 13-09-2007 |
| | | | US 2008132749 A1 | | 05-06-2008 |
| | | | WO 2004078025 A2 | | 16-09-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016193412 A
- WO 2017036695 A

**Non-patent literature cited in the description**

- **B. A. ACHRAF ; A. B. KOUKI ; C. HUNG.** *Power Approaches for Implantable Medical Devices,* 2015
- **J. LEE ; J. JANG ; Y.-K. SONG.** A review on wireless powering schemes for implantable microsystems in neural engineering applications. *Biomed Eng Letters,* 2016, vol. 6, 205-215
- **A. KIM ; M. OCHOA ; R. RAHIM ; B. ZIAIE.** New and Emerging Energy Sources for Implantable Wireless Microdevices. *IEEE: SPECIAL SECTION ON NANO-BIOSENSORS,* 2014
- **K. N. BOCAN ; E. SEJDI'C.** *Adaptive Transcutaneous Power Transfer to Implantable Devices: A State of the Art Review,* 2016, vol. 16, 393